# EUROPÄISCHE PATENTSCHRIFT

(11) **EP 1 435 971 B1**
(45) Veröffentlichungstag und Bekanntmachung des Hinweises auf die Patenterteilung: **02.08.2006**
(21) Anmeldenummer: 02781213.0
(22) Anmeldetag: 04.10.2002
(51) Int. Cl.: A61K 31/685, A61P 25/00, A23L 1/30

(54) **VERWENDUNG VON PHOSPHATIDYLSERIN ZUR BEHANDLUNG DES AUFMERKSAMKEITS-DEFIZIT-SYNDROMS (ADHS)**
UTILIZATION OF PHOSPHATIDYLSERINE IN THE TREATMENT OF ATTENTION DEFICIT SYNDROME (ADHS)
UTILISATION DE PHOSPHATIDYLSERINE POUR TRAITER LE SYNDROME D'HYPERACTIVITE AVEC DEFICIT D'ATTENTION (ADHS)

(30) Priorität: 05.10.2001 DE 10149108
(43) Veröffentlichungstag der Anmeldung: 14.07.2004
(73) Patentinhaber: Bioghurt Biogarde GmbH & Co. KG, 85354 Freising (DE)
(72) Erfinder: JÄGER, Ralf, 85354 Freising (DE); BÖKENKAMP, Dirk, 59368 Werne (DE)
(74) Vertreter: Dey, Michael
(86) Internationale Anmeldenummer: PCT/EP2002/011124
(87) Internationale Veröffentlichungsnummer: WO 2003/030914

(56) Entgegenhaltungen:
- EP-A- 1 275 399
- WO-A-01/78704
- KIDD PM.: "ATTENTION DEFICIT/HYPERACTIVITY DISORDER (ADHD) IN CHILDREN" ALTERNATIVE MEDICINE REVIEW, Bd. 5, Nr. 5, 2000, Seiten 402-428, XP008012816

## Beschreibung

Gegenstand der vorliegenden Erfindung ist die Verwendung von Phosphatidylserin, Lyso-Phosphatidylserin oder/und einem physiologisch verträglichen Salz davon sowie die Verwendung von Phosphatidylserin, Lyso-Phosphatidylserin oder/und einem physiologisch verträglichen. Salz davon in Kombination mit Decosahexaensäure (DHA) zur Behandlung des Aufmerksamkeits-Defizit-Syndroms (ADHS).

Unter dem Aufmerksamkeits-Defizit-Syndrom (ADHS; Attention deficit/Hyperactivity disorder) versteht man ein klinisches Syndrom, das durch erhebliche Beeinträchtigungen der Konzentrations- und Daueraufmerksamkeitsfähigkeit, Störungen der Impulskontrolle sowie eine fakultative motorische Hyperaktivität bzw. Unruhe gekennzeichnet ist. In der Vergangenheit wurde dieses Syndrom auch als "Hyperkinetisches Syndrom" oder "minimale cerebrale Dysfunktion" bezeichnet.

Das Krankheitsbild des ADHS ist mit neurobiologisch definierten Veränderungen im Hirnstoffwechsel bzw. im Bereich der Neurotransmitter verbunden, sodass es sich dabei primär um ein neurologisches Krankheitsbild handelt, das zu erheblichen psychischen Folgen führen kann (Swanson JM, Sergeant JA, Taylor E, Sonuga-Barke EJ, Jensen PS, Cantwell DP, Attention-deficit hyperactivity disorder and hyperkinetic disorder, Lancet 1998; 351:429-33).

In den vergangenen Jahren wurde aufgrund neuerer Untersuchungen auch ein spezielles Aufmerksamkeits-Defizit-Syndrom ohne Anzeichen motorischer Hyperaktivität als sogenannter "inattentive type" abgegrenzt (Lahey BB; Carlson CL, Validity of the diagnostic category of attention deficit disorder without hyperactivity: A review of the literature, Journal of Learning Disabilites 1991; 24:110-120). Für Erwachsene, bei denen nicht mehr alle typischen Symptome des Kindesalters nachzuweisen sind, wird in der geltenden DSM-("Diagnostic and Statistical Manual of Mental Disorders")-Klassifikation die Zusatzbezeichnung "in partieller Remission" oder auch "Hyperkinetisches Syndrom - Residualtyp" vorgeschlagen.

Bislang ist in Deutschland die Symptomatik des Aufmerksämkeits-Defizit-Syndroms bei Erwachsenen noch weitgehend unbeschrieben, öbwohl das sogenannte Hyperaktivitätssyndröm oder auch Hyperkinetische Syndrom zu den häufigsten kinder- und jugendpsychiatrischen Störungen zählt. Dabei haben Verlaufsuntersuchungen bei diesen Kindern eine bis in das Erwachsenenalter anhaltende schwere Beeinträchtigung gezeigt, die unter anderem mit dem Auftreten von aggressiven Verhaltensstörungen bzw. mit antisozialen Persönlichkeitsstörungen, Suchterkrankungen sowie affektiven Störungen korreliert (Mannuzza S, Klein RG, Bessler A, Malloy P, LaPadula M, Adult outcome of hyperactive boys, Arch General Psychiatry 1993; 50:565-76).

Ein umfassender Überblick über die Behandlung von ADHS wurde von KIDD PM vorgestellt (Kidd PM, Attention Deficit/Hyperactivity Disorder (ADHA) in Children, Alternative Medicine Review, Bd. 5, Nr. 5, 2000, 402-428).

Der Anteil des ADHS in der Bevölkerung wird heute bei Kindern im Grundschulalter bzw. Jugendlichen auf 3 - 4 % bzw. 2 % geschätzt. Der geschätzte Anteil aller Kinder, Jugendlichen und Erwachsenen, die an ADHS leiden, beträgt 2-10 %. Im Gegensatz zu der Ansicht, dass sich im Erwachsenenalter die Symptomatik verliert, sind bei bis zu 60 % der ehemals betroffenen Kinder noch als Jugendliche deutliche Beeinträchtigungen festzustellen.

Bei ADHS handelt es sich meist um eine zunächst rein biologische Prädisposition, die nach Ausprägung und den persönlichen Lebensumständen der Betroffenen jedoch zu sehr unterschiedlichen Auswirkungen führen kann.

Nach aktuellen Untersuchungen ist davon auszugehen, dass bei Kenntnis der für dieses Krankheitsbild typischen Symptomatik bei Patienten, deren Symptomatik auf eine reine Angststörung oder eine impulsive, dissoziale, hysterisch exzentrische oder depressive Störung zurückgeführt wurde, zumindest als zusätzliche Diagnose ein bis in das Erwachsenenalter persistierendes Hyperkinetisches Syndrom gestellt werden muss.

Als Grundstörungen des Aufmerksamkeits-Defizit-Syndroms, bei dem die Betroffenen zumeist nicht "unaufmerksam" sind, sondern vielmehr mehreren inneren oder äußeren Wahrnehmungen bzw. Gedanken gleichzeitig zu folgen versuchen, sind Störungen der selektiven Aufmerksamkeit typisch. Insgesamt ist bei den Betroffenen die Selbstkontrollfähigkeit stark gestört, woraus bei verminderter Hemmung ein Versagen wesentlicher Handlungsfunktionen im Bereich der Wachheit, Gefühlsregulation, kognitiver Funktionen, Gedächtnis und Handlungsplanung resultiert.

Üblicherweise können Menschen auch bei als unangenehm oder langweilig einzustufenden Aufgaben eine gewisse Motivation und Ausdauer aufbringen, wenn als Konsequenz daraus etwas Positives zu erwarten ist. Bei Personen mit ADHS ist es umgekehrt: Es ist zunächst eine Stimulierung oder Aktivierung nötig, damit überhaupt eine Aktivität begonnen werden kann.

Aus einer verminderten Hemmfunktion bei ADHS resultiert klinisch einerseits eine besondere Übererregbarkeit bzw. Hypersensibilität sowie eine Störung der Impulskontrolle, wodurch sich auch eine verstärkte Ablenkbarkeit bzw. eine verminderte Aufmerksamkeits- und Konzentrationsfähigkeit ergibt.

Nach den derzeit geltenden Diagnosekriterien sollten sich Symptome des ADHS bereits vor dem 7. Lebensjahr manifestiert haben.

Häufig treten bei ADHS-Kindern bereits in der frühen Kindheit geringfügige Anomalien, Entwicklungsverzögerungen, wie z.B. beim Laufen oder Sprechen bzw. Störungen der Wahrnehmung oder Feinmotorik, auf. Auch werden sogenannte "soft-signs" in Form feinneurologischer Besonderheiten wie Muskeltonusabweichungen oder Reflex- und Sensibilitätsasymmetrien beschrieben, die unter anderem zu einem auffällig festen Aufdrücken oder Verkrampfungen bei der Schreibschrift mit einem schlechten Schriftbild führen. Besonders häufig tritt auch Bettnässen nach dem 7. Lebensjahr auf.

Gemäß P. H. Wender (Attention Deficit Hyperactivity Disorder in Adults, Oxford University Press, 1995) lässt sich das Hyperaktivitätssyndrom im Kindesalter wie folgt zusammenfassen:
- Hyperaktivität, bzw. über das durchschnittliche Maß unruhige Kinder, nervös, im Rededrang nicht zu bremsen, ruhelos.
- Aufmerksamkeits-Defizitebzw.verminderteAufmerksamkeits-Spanne, vermehrte Ablenkbarkeit.
- Das betroffene Kind erreicht nicht sein volles Leistungspotänzial, das ihm zugetraut wird.
- "Könnte, wenn er/sie nur wollte". Vergesslichkeit, Probleme bei Kettenaufgaben und beim Anweisungen befolgen und Aufgaben abschließen.

Als Leitsymptom im Erwachsenenalter steht häufig ein Gefühl, nicht alle Ziele zu erreichen, die man sich gesteckt hat bzw. eine auffällige Leistungsschwäche im Vordergrund. Typische Symptome für ein ADHS im Erwachsenenalter sind beispielsweise auch kognitive Auffälligkeiten, veränderte Daueraufmerksamkeitsspanne, Störungen des Arbeitsgedächtnisses, Hypersensitivität und Impulsivität, geringe Stresstoleranz und Impulsivität, aber auch Suchtverhalten oder hochgradige Stimulierungen in Form beispielsweise eines "Adrenalin-Kicks", die "klassische" Hyperaktivität und Unfähigkeit zur Entspannung.

Bei der Substanzklasse der Phosphatidylserine handelt es sich um Phospholipide, die natürlicherweise im Körper vorkommen und die früher in der Gruppe der Lysolecithine zusammengefasst wurden.

Phosphatidylserin unterstützt zahlreiche Funktionen der Körperzellen und tritt in natürlich erhöhten Konzentrationen im Gehirn auf, wo es die äußerst empfindlichen Funktionen der Nervenzellen und der mit ihnen verbundenen Zellen unterstützt. Phosphatidylserin ist eine gut untersuchte Substanz, die bei Lebewesen aller Lebensabschnitte zu deren Wohlbefinden und Gesundheit beiträgt. Es zeigt vor allem eine sogenannte' Anti-Aging-Wirkung. Phosphatidylserin" wirkt gegen Stress, antidepressiv und in gewisser Weise sogar verjüngend. Mit seinen gedächtnissteigernden Fähigkeiten, seiner gemütsaufhellenden Wirkung sowie seiner Eigenschaft, Stresshormone im Blut zu senken, gilt Phosphatidylserin als der wichtigste aller Nährstoffe für das Gehirn.

Als Nahrungsergänzungsmittel wirkt Phosphatidylserin, wie im Übrigen auch die anderen Phospholipide, nicht nur in direkter Weise günstig auf die Gesundheit. Phosphatidylserin kann gleichermaßen die Aufnahme zahlreicher anderer Nahrungsstoffe oder Nahrungsergänzungsmittel verbessern oder gemeinsam mit diesen synergistisch wirken. Dies wurde in klinischen Doppelblindstudien nachgewiesen.

Als Phospholipid ist Phosphatidylserin ein bedeutender Baustein der Zellmembranen. Diese sehr dünnen, flexiblen, dreidimensionalen Strukturen stellen mit ihren Phospholipid-Anteilen eine wichtige Matrix für Enzymsysteme dar, die unter anderem den Energiehaushalt der Zellen regeln bzw. aufrechterhalten und so die reibungslose Zusammenarbeit der Körperzellen gewährleisten.

Von Phosphatidylserin ist des Weiteren bekannt, dass es das Gehirn bei der Energieerzeugung unterstützt, dass es günstig auf die Zell/Zellverbindungen (Synapsen) wirkt, dass es die Wirkung chemischer Transmittersubstanzen, wie Acetylcholin, Dopamin, Noradrenalin und Serotonin verstärkt, wodurch bessere kognitive Fähigkeiten des Gehirns, wie z.B. Konzentration, Lernvermögen, Kurzzeitgedächtnis und Wort-Erinnerungen resultieren.

Phosphatidylserin stellt auch einen bedeutenden Wirkstoff gegen Alterungsvorgänge im Gehirn dar, was zwischenzeitlich durch etwa 18 Doppelblindstudien und über 40 publizierte Humanstudien belegt worden ist. Zudem wird Phosphatidylserin die Fähigkeit zugesprochen, Gedächtnisvertuste, die mit Alterungsprozessen zusammenhängen, zumindest teilweise zu revertieren.

Zur Therapie des ADHS kann neben anderen Therapieformen auch eine medikamentöse Behandlung durchgeführt werden. Im Stand der Technik wird ein erster Versuch zur Behandlung von ADHS-Patienten mit Phosphatidylserin beschrieben. In diesem Versuch wurden zunächst geringe Dosierungen von 100 mg pro Tag über einen Zeitraum von 2 Monaten verwendet. Diese Behandlung bewirkte keine eindeutige Verbesserung der Symptome. Daraufhin wurden über einen Zeitraum von 4 Monaten höhere Dosierungen verabreicht (200 bis 300 mg täglich, bei schweren Symptomen bis zu 500 mg täglich). Diese Behandlung mit hohen Dosierungen zeigte bei 25 von 27 Propanden im Alter von 3 bis 19 Jahren, die alle typische ADHS-Symptome zeigten, eine positive Wirkung. Bei einem Probanden (7 Jahre, weiblich) konnte lediglich eine leichte Besserung erzielt werden, bei einem weiteren Probanden (15 Jahre, weiblich) war keine klare Aussage über eine Besserung möglich (Dr. Parris Kidd, Clear Viewpoint towards ADHD: PS, Lecture presented at the 11th Food Design Show, Tokyo Big Sight, Tokyo, Japan, September 20th, 2000). Bei diesem Versuch traten keine Nebenwirkungen mit gleichzeitig verabreichten Arzneimitteln auf, jedoch waren die verwendeten Dosierungen relativ hoch.

Der vorliegenden Erfindung lag somit die Aufgabe zugrunde, ein effizientes Mittel auf der Basis von Phosphatidylserin zur Behandlung des Aufmerksamkeits-Defizit-Syndroms (ADHS) bereitzustellen, wobei bei einer effizienten Dosierung eine gute physiologische Verträglichkeit und eine gute Compliance erreicht werden soll und keine Nebenwirkungen auftreten dürfen.

Eine weitere Aufgabe der Erfindung lag in der Bereitstellung weiterer effizienter Dosierungsformen im Hinblick auf die Menge bzw. Dauer der Verabreichung von Phosphatidylserin bei der Behandlung von ADHS.

Gelöst wurde diese Aufgabe durch die Verwendung von Phosphatidylserin, Lyso-Phosphatidylserin oder/und einem physiologisch verträglichen Salz davon alleine oder in Kombination mit Decosahexaensäure (DHA). Der Begriff Phosphatidylserin (PS), wie er hierin verwendet wird, umfasst alle der Substanzklasse der Phosphatidylserine angehörenden Verbindungen, einschließlich der Lyso-Verbindungen und der entsprechenden Salze. Beispiele hierfür sind u.a. Phosphatidyl-L-Serin oder Lyso-Phosphatidyl-L-Serin.

Überraschenderweise wurde nun festgestellt, dass PS bei der Verwendung zur Behandlung des ADHS in Kombination mit anderen Substanzen synergistische Effekte zeigt. Die vorliegende Erfindung betrifft somit die Verwendung von Phosphatidylserin (PS), Lyso-Phosphatidylserin oder/und einem physiologisch verträglichen Salz davon in Kombination mit DHA zur Herstellung eines Mittels zur Behandlung des Aufmerksamkeits-Defizit-Syndroms (ADHS). Der Begriff "Behandlung" wie er hierin verwendet wird, umfasst sowohl die Therapie als auch die Prophylaxe des ADHS.

Durch die erfindungsgemäße Verwendung von PS ist einerseits eine Reduzierung der PS-Dosis, andererseits eine Verringerung des Behandlungszeitraums möglich. Zudem ergab die erfindungsgemäße Verwendung von Phosphatidylserin, dass dessen bekannt gute Verträglichkeit auch in hohen Dosen und über einen längeren Supplementations-Zeitraum keinerlei Einschränkung erfährt, dass die Compliance mit Phosphatidylserin keine Schwierigkeiten bereitet und bei den Probanden mit ADHS auch keine Gewöhnungseffekte auftreten. Außerdem entfaltet PS bei der erfindungsgemäßen Verwendung seine positive Wirkung bei ADHS in signifikant stärkerem Ausmaß als die bislang bekannten Medikamente.

Bei der erfindungsgemäßen Verwendung einer Kombination von Phosphatidylserin (PS) und DHA hat es sich als günstig erwiesen, wenn das Phosphatidylserin in Tagesdosen von 20 bis 1000 mg, bevorzugt von 50 bis 600 mg verabreicht wird.

Für die DHA sind Tagesdosen von 1 bis 10.000 mg, besonders von 5 bis 5000 mg und insbesondere von 50 bis 1000 mg geeignet.

Dabei ist eine Verabreichung über einen Zeitraum von mindestens 7 Tagen und bevorzugt nicht länger als 6 Monate geeignet, wobei die jeweiligen Supplementationszeiträume nach Pausen oder/und Neueinstellung der Tagesdosis ohne Weiteres und völlig problemlos wiederholt werden können. Der Zeitraum von 7 Tagen muss hierbei nicht unbedingt eine zusammenhängende Abfolge von Tagen sein.

Geeignete weitere Zusatzmittel für die erfindungsgemäße Verwendung umfassen Antioxidantien, essenzielle Fettsäuren, Mineralstoffe, Aminosäuren, Gemütsaufheller, Phospholipide oder/und Gemische davon. Die genannten Zusatzmittel wirken vor allem positiv auf die Gehirnfunktion: Antioxidantien schützen insbesondere Gewebe oder Organe, die intensiv mit Sauerstoff versorgt werden; essenzielle Fettsäuren und Phospholipide unterstützen bzw. erhalten die Funktion von Zellmembranen und Nervenzellen bzw. -gewebe, wie sie insbesondere im Gehirn vorkommen; Mineralstoffe, insbesondere Magnesium und Calcium, spielen ebenfalls eine bedeutende Rolle im neuronalen Geschähen.

Bevorzugte Antioxidantien sind beispielsweise Bioflavonoide, Vitamin E, Vitamin C, α-Liponsäure oder/und Carotinoide. ω-3- und ω-6-Fettsäuren und besonders bevorzugt Docosahexaensäure (DHA) und γ-Linolensäure (GLA) sind Beispiele für geeignete essenzielle Fettsäuren. Geeignete Aminosäuren sind z.B. essenzielle Aminosäuren oder/und Aminosäure-Derivate, wie z.B. Kreatin. Magnesium und Calcium sind Beispiele für bevorzugte Mineralstoffe. Besonders bevorzugt werden als Gemütsaufheller z.B. Johanniskraut-Extrakte, Kava-Kava oder/und Gemische davon verwendet. Bevorzugte Phospholipide sind beispielsweise Phosphatidylcholin (Lecithin), Phosphatid-Säure, Phosphatidylethanolamin oder/und Phosphatidylinosit.

Überraschend wurde bei der erfindungsgemäßen Verwendung von Phosphatidylserin festgestellt, dass die Verwendung von Phosphatidylserin (PS) in geringen Dosierungen über einen längeren Zeitraum und in höheren Dosierungen über einen kurzen Zeitraum zur Behandlung von ADHS Symptomen entgegen dem bisherigen Stand der Technik hervorragend geeignet ist.

Auch diese erfindungsgemäße Verwendung von Phosphatidylserin ergab eine gute Verträglichkeit, auch in hohen Dosen und über einen längeren Supplementationszeitraum hinweg, eine gute Compliance und zudem traten auch hier keine Gewöhnungseffekte der ADHS-Patienten auf.

Es hat es sich als sehr günstig erwiesen, wenn die längere Phosphatidylserin-Gabe (über mehr als 2 Monate) bevorzugt in Tagesdosen von 50 bis 150 mg und mehr bevorzugt in Tagesdosen von 100 bis 150 mg erfolgt. Bei kurzzeitiger Gabe von Phosphatidylserin über einen Zeitraum von maximal 2 Monaten haben sich Tagesmengen von 150 bis 600 mg und bevorzugt von 250 bis 450 mg als besonders geeignet erwiesen.

Phosphatidylserin wird als körpereigene Substanz im Allgemeinen sehr rasch und vollständig verstoffwechselt und entfaltet daher bereits nach kurzer "Anflutzeit" seine gute Wirkung. Dennoch werden bei der ADHS-Behandlung mit PS in den Ausführungsformen der vorliegenden Erfindung 7 Tage, die nicht unbedingt zusämmenhängen müssen, als minimale Behandlungsdauer betrachtet. Als Obergrenze für die regelmäßige Einnahme von Phosphatidylserin bei ADHS sind erfindungsgemäß 6 Monate anzusehen, doch können die Supplementationszeiträume nach Pausen oder/und Neueinstellung der Tagesdosis ohne weiteres und völlig problemlos mehrfach wiederholt werden.

Für die erfindungsgemäße Verwendung von Phosphatidylserin(-Derivaten) zur Behandlung von ADHS hat sich ein Probanden-Klientel als geeignet erwiesen, das sich im Alter zwischen 2 und 20 Jahren befindet, wobei sich nicht zuletzt zur Vorbeugung von späteren irreparablen Dauersymptomen und im Interesse einer problemlosen Compliance ein Alter zwischen 3 und 10 Jahren als besonders geeignet erwiesen hat.

Die Verwendung des Phosphatidylserins (PS), dessen geeigneter Derivate, des Lyso-Phosphatidylserins oder/und eines physiologisch verträglichen Salzes davon bei ADHS kann erfindungsgemäß für alle Ausführungsformen sowohl in festen als auch in flüssigen Formulierungen erfolgen.

Als feste Formulierungen sind beispielsweise Pulver-, Kau-, Lutsch- und Brausetabletten, Dragees und Kapseln sowie - in Anbetracht des meist geringen Alters der bevorzugten Probanden Bonbons oder Schokoriegel geeignet. Als flüssige Formulierungen sind z.B. Säfte und Softdrinks besonders geeignet.

Erfindungsgemäß kann die Formulierung weiterhin physiologisch verträgliche oder/und physiologisch wirksame Zusätze oder/und Formulierungshilfsmittel enthalten. Geeignete physiologisch verträgliche oder/und physiologisch wirksame Zusätze für die erfindungsgemäße Verwendung sind beispielsweise Antioxidantien, Vertreter der essenziellen Fettsäuren, Antidepressiva (z.B. Gemütsaufheller, bevorzugt pflanzlichen Ursprungs wie Johanniskraut oder Kava Kava), Probiotica, verschiedene Phospholipide von PS oder die natürliche Gehirnsubstanz Dimethylaminoethanol (DMAE) sowie Alkohole, Zucker, Vitamine, Spurenelemente, Aminosäuren, Neurotransmitter, Stimulanzien, Farb- und Aromastoffe. Darüber hinaus können auch additiv oder synergistisch wirkende Kombinationen von Phosphatidylserin mit den bislang bekannten oder anderen bei ADHS wirksamen Medikamenten verwendet werden. Wenn sich synergistische Effekte ergeben, können die jeweiligen effektiven PS-Dosen vorteilhafterweise gesenkt werden, wobei die jeweilige Tagesmenge dann insbesondere bei Kindern und Jugendlichen auf deren Körperindizes, wie Größe und Gewicht abgestimmt werden kann.

Geeignete Formulierungshilfsmittel für die erfindungsgemäße Verwendung sind beispielsweise Kohlenhydrate (z.B. Methylcellulose), SiO₂, Stearate, Lösevermittler, Aromastoffe, Konservierungs- und Trennmittel sowie Texturantien.

Aufgrund seiner guten physiologischen Verträglichkeit und signifikanten Wirkung beim Aufmerksamkeits-Defizit-Syndrom ist Phosphatidylserin (PS) im Rahmen der vorliegenden Erfindung insbesondere als Therapeutikum oder Nahrungsergänzungsmittel sehr gut geeignet, wobei vor allem im letzten Fall die Dosierung gering gehalten und die Verabreichung auch über längere Zeiträume erfolgen kann.

Die nachfolgenden Beispiele 1 bis 4 verdeutlichen die gute Wirksamkeit und Compliance bei der erfindungsgemäßen Verwendung von Phosphatidylserin (PS) oder von Phosphatidylserin (PS) in Kombination mit einem Zusatzmittel.

### Beispiele

Probanden einer offenen Studie (Beispiel 1 bis 4) wurden mit Hilfe der Wender Otah Rating Scales (WURS); aufgrund schulischer Leistungen sowie klinischer und physiologischer Tests ausgewählt.

Die Auswertung erfolgte bei kurzzeitiger Supplementierung nach 4, 6 und 8 Wochen, bei längerer Supplementierung nach 2, 4 und 6 Monaten. Die Probanden wurden auf messbare Unterschiede hinsichtlich Konzentration, Aufmerksamkeit, Impulsivität, des Aggressionspotenzials und der Hyperaktivität untersucht. Probanden, Eltern und Lehrer wurden durch Fragebogen in die Evaluierung einbezogen.

### Beispiel 1

Einer 9-jährigen Schülerin wurden über einen Zeitraum von 9 Wochen jeden zweiten Tag 150 mg Phosphatidylserin, sowie 100 mg einer Mischung aus Vitamin E, Vitamin C, DHA und GLA verabreicht. Positive Tendenzen waren bereits nach 4 Wochen, eine eindeutige positive Wirkung nach 6 Wochen feststellbar. Die Aufmerksamkeit, Konzentration und Gedächtnisleistungen sowie die schulischen Leistungen besserten sich deutlich.

### Vergleichs-Beispiel 2

Einem 14-jährigem Schüler wurden über einen Zeitraum von 7 Wochen 3 x täglich 200 mg Phosphatidylserin verabreicht. Nach Ablauf der Supplementierung war ein merklicher Rückgang der Hyperaktivität zu beobachten; der Proband berichtete zudem über eine Verbesserung des allgemeinen Empfindens.

### Vergleichs-Beispiel 3

Einem 8-jährigen Schüler wurden über einen Zeitraum von 6 Monaten pro Tag 100 mg Phosphatidylserin verabreicht. Ab dem dritten Monat wurde eine Verbesserung des allgemeinen Wohlbefindens sowie eine Aufhellung der Stimmungslage beobachtet. Die Aufmerksamkeit, Konzentration und Gedächtnisleistungen sowie die allgemeinen schulischen Leistungen besserten sich deutlich.

### Beispiel 4

An einen 11-jährigen Schüler wurden 8 Wochen lang pro Tag 400 mg Phosphatidylserin in Kombination mit 100 mg einer DHA, Johanniskraut und Vitamin E und C enthaltenden Mischung verabreicht. Eine deutliche Verbesserung der Stimmungslage, verringerte Aggression sowie verbesserte schulische Leistungen konnten bereits nach kurzer Supplementierung beobachtet werden.

## Patentansprüche

1. Verwendung von Phosphatidylserin (PS), Lyso-Phosphatidylserin oder/und einem physiologisch verträglichen Salz davon in Kombination mit Decosahexaensäure (DHA) zur Herstellung eines Mittels zur Behandlung des Aufmerksamkeits-Defizit-Syndroms (ADHS).

2. Verwendung zur Herstellung eines Mittels nach Anspruch 1,
**dadurch gekennzeichnet,**
**dass** das Mittel Phosphatidylserin, Lyso-Phosphatidylserin oder/und ein physiologisch verträgliches Salz davon zur Verabreichung in einer Tagesdosis von 20 bis 1000 mg enthält.

3. Verwendung zur Herstellung eines Mittels nach Anspruch 2,
**dadurch gekennzeichnet,**
**dass** das Mittel Phosphatidylserin, Lyso-Phosphatidylserin oder/und ein physiologisch verträgliches Salz davon zur Verabreichung in einer Tagesdosis von 50 bis 600 mg enthält.

4. Verwendung zur Herstellung eines Mittels nach einem der Ansprüche 1 bis 3,
**dadurch gekennzeichnet,**
**dass** das Mittel Decosahexaensäure zur Verabreichung in einer Tagesdosis von 1 bis 10000 mg enthält.

5. Verwendung zur Herstellung eines Mittels nach Anspruch 4,
**dadurch gekennzeichnet,**
**dass** das Mittel Decosahexaensäure zur Verabreichung in einer Tagesdosis von 5 bis 5000 mg enthält.

6. Verwendung zur Herstellung eines Mittels nach Anspruch 4 oder 5,
**dadurch gekennzeichnet,**
**dass** das Mittel Decosahexaensäure zur Verabreichung in einer Tagesdosis von 50 bis 1000 mg enthält.

7. Verwendung zur Herstellung eines Mittels nach einem der Ansprüche 1 bis 6,
**dadurch gekennzeichnet,**
**dass** das Mittel für eine Verabreichung für maximal 2 Monate mit einer Tagesdosis von 150 bis 600 mg vorgesehen ist.

## Claims

1. Use of phosphatidylserine (PS), lyso-phosphatidylserine or/and a physiologically acceptable salt thereof in combination with decosahexaenoic acid (DHA) to produce an agent for treating the attention deficit syndrome (ADHS).

2. Use to produce an agent as claimed in claim 1,
**characterized in that**
the agent contains phosphatidylserine, lyso-phosphatidylserine or/and a physiologically acceptable salt thereof for administration in a daily dose of 20 to 1000 mg.

3. Use to produce an agent as claimed in claim 2,
**characterized in that**
the agent contains phosphatidylserine, lyso-phosphatidylserine or/and a physiologically acceptable salt thereof for administration in a daily dose of 50 to 600 mg.

4. Use to produce an agent as claimed in one of the claims 1 to 3,
**characterized in that**
the agent contains decosahexaenoic acid for administration in a daily dose of 1 to 10000 mg.

5. Use to produce an agent as claimed in claim 4,
**characterized in that**
the agent contains decosahexaenoic acid for administration in a daily dose of 5 to 5000 mg.

6. Use to produce an agent as claimed in claim 4,
**characterized in that**
the agent contains decosahexaenoic acid for administration in a daily dose of 50 to 1000 mg.

7. Use to produce an agent as claimed in one of the claims 1 to 6,
**characterized in that**
the agent is intended to be administered for a maximum of 2 months with a daily dose of 150 to 600 mg.

## Revendications

1. Utilisation de phosphatidylsérine (PS), de lyso-phosphatidylsérine et/ou d'un de leurs sels physiologiquement compatible en combinaison avec l'acide docosahexaénoïque (DHA) pour préparer un remède destiné à traiter le syndrome de déficit d'attention (ADHS).

2. Utilisation pour préparer un remède selon la revendication 1, **caractérisée en ce que** le remède contient de la phosphatidylsérine (PS), de la lyso-phosphatidylsérine et/ou un de leurs sels physiologiquement compatible à administrer à une dose journalière de 20 à 1 000 mg.

3. Utilisation pour préparer un remède selon la revendication 2, **caractérisée en ce que** le remède contient de la phosphatidylsérine (PS), de la lyso-phosphatidylsérine et/ou un de leurs sels physiologiquement compatible à administrer à une dose journalière de 50 à 600 mg.

4. Utilisation pour préparer un remède selon l'une des revendications 1 à 3, **caractérisée en ce que** le remède contient de l'acide docosahexaénoïque à administrer à une dose journalière de 1 à 10 000 mg.

5. Utilisation pour préparer un remède selon la revendication 4, **caractérisée en ce que** le remède contient de l'acide docosahexaénoïque à administrer à une dose journalière de 5 à 5 000 mg.

6. Utilisation pour préparer un remède selon la revendication 4 ou 5, **caractérisée en ce que** le remède contient de l'acide docosahexaénoïque à administrer à une dose journalière de 50 à 1 000 mg.

7. Utilisation pour préparer un remède selon l'une des revendications 1 à 6, **caractérisée en ce que** le remède est prévu pour être administré pendant 2 mois au plus à une dose journalière de 150 à 600 mg.
